(19) Europäisches Patentamt / European Patent Office / Office européen des brevets

(11) **EP 3 145 405 B1**

(12) **FASCICULE DE BREVET EUROPEEN**

(45) Date de publication et mention de la délivrance du brevet:
**01.11.2023 Bulletin 2023/44**

(21) Numéro de dépôt: **15728072.8**

(22) Date de dépôt: **19.05.2015**

(51) Classification Internationale des Brevets (IPC):
*A61B 5/103* (2006.01)    *G02C 13/00* (2006.01)
*G02C 7/00* (2006.01)    *A61B 5/107* (2006.01)
*A61B 5/16* (2006.01)

(52) Classification Coopérative des Brevets (CPC):
**G02C 13/005; A61B 5/1077; A61B 5/1079;
A61B 5/16; A61B 5/163**

(86) Numéro de dépôt international:
**PCT/FR2015/051316**

(87) Numéro de publication internationale:
**WO 2015/177461 (26.11.2015 Gazette 2015/47)**

(54) **PROCÉDÉ DE DÉTERMINATION D'AU MOINS UN PARAMÈTRE COMPORTEMENTAL**

VERFAHREN ZUR BESTIMMUNG VON MINDESTENS EINEM VERHALTENSPARAMETER

METHOD OF DETERMINING AT LEAST ONE BEHAVIOURAL PARAMETER

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.05.2014 FR 1454548**

(43) Date de publication de la demande:
**29.03.2017 Bulletin 2017/13**

(73) Titulaire: **Essilor International
94220 Charenton-le-Pont (FR)**

(72) Inventeurs:
• **BARANTON, Konogan
94227 Charenton-le-Pont Cedex (FR)**

• **ESCALIER, Guilhem
94227 Charenton-le-Pont Cedex (FR)**
• **GAYAT, Sébastien
94227 Charenton-le-Pont Cedex (FR)**

(74) Mandataire: **Jacobacci Coralis Harle
32, rue de l'Arcade
75008 Paris (FR)**

(56) Documents cités:
WO-A1-02/09025    WO-A1-2014/016502
FR-A1- 2 974 722    FR-A1- 2 987 918

## Description

### DOMAINE TECHNIQUE AUQUEL SE RAPPORTE L'INVENTION

La présente invention concerne de manière générale un procédé de détermination d'au moins un paramètre comportemental d'un porteur en vue de la conception d'une lentille ophtalmique pour une monture de lunettes de ce porteur.

### ARRIERE-PLAN TECHNOLOGIQUE

La conception et la fabrication d'une lentille ophtalmique adaptée à un porteur et à une monture de lunettes choisie nécessite actuellement une ou plusieurs prises de mesure chez l'opticien.

Ces prises de mesure sont souvent biaisées par le comportement non naturel du porteur chez l'opticien. Le cadre du magasin de l'opticien est souvent éloigné du cadre de vie et donc du contexte de l'utilisation habituelle du porteur. En outre, les mesures réalisées chez l'opticien doivent être réalisées très rapidement.

Les mesures réalisées en magasin présentent donc une imprécision liées aux conditions peu naturelles de la mesure.

On connaît également des systèmes de prise de mesure déportés hors du magasin de l'opticien pour les achats de paires de lunettes sur internet.

Ces systèmes permettent alors une prise de mesure dans des conditions plus proches de celles du quotidien du porteur.

Cependant, les mesures réalisées par ces systèmes connus se limitent uniquement à des paramètres géométrico-morphologiques, tels que la valeur de l'écart pupillaire. Ces systèmes connus ne permettent pas de déterminer des paramètres comportementaux du porteur, c'est-à-dire liés au comportement visuel du porteur, comme par exemple la distance de lecture ou le coefficient oeil-tête.

La détermination de ces paramètres comportementaux est plus complexe et les mesures associées sont actuellement uniquement faisables chez l'opticien. Des procédés de détermination sont connus des documents WO2014/016502 A1, WO02/09025 A1, FR 2987 918 A1 et FR 2 974 722 A1.

### OBJET DE L'INVENTION

Afin de remédier aux inconvénients précités de l'état de la technique, la présente invention propose un nouveau procédé de détermination d'au moins un paramètre comportemental du porteur, grâce auquel il est possible de déterminer précisément ce paramètre dans des conditions naturelles pour le porteur.

Plus particulièrement, on propose selon l'invention un procédé de détermination d'au moins un paramètre comportemental d'un porteur en vue de la conception d'une lentille ophtalmique pour une monture de lunettes de ce porteur, comportant les étapes énoncées dans la revendication 1.

On peut ainsi tenir compte de la position et/ou de l'orientation de la tête du porteur dans cette posture naturelle lors de la détermination du paramètre comportemental.

Un exemple de paramètre comportemental est constitué par le port de tête du porteur (défini par exemple par un angle d'abaissement et/ou un angle de roulis). D'autres exemples sont donnés dans la description qui suit.

Chaque set de données peut comprendre des données stockées dans différents fichiers. Un set de données peut par exemple regrouper les données d'un fichier correspondant à une image et des méta-données stockées dans un autre fichier.

D'autres caractéristiques du procédé conforme à l'invention sont énoncées dans les revendications 1 à 17.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 est un diagramme bloc représentant de manière schématique le procédé selon l'invention,
- la figure 2 représente schématiquement une possibilité de mise en oeuvre de l'étape a) du procédé.

L'invention concerne un procédé de détermination d'au moins un paramètre comportemental d'un porteur en vue de la conception d'une lentille ophtalmique pour une monture de lunettes de ce porteur.

Ce paramètre comportemental est l'un des suivants :

- la distance de lecture, c'est-à-dire la distance séparant les yeux du porteur du support portant le texte lu ou la cible visuelle fixée du regard par le porteur,
- le rapport des déplacements angulaires de l'oeil et de la tête, c'est-à-dire le rapport entre l'amplitude du déplacement d'un oeil du porteur selon une direction déterminée et l'amplitude maximale du déplacement de cet oeil pendant la tâche de lecture, aussi appelé coefficient oeil-tête,

- la posture statique ou dynamique adoptée pour l'activité habituelle concernée, par exemple la posture de la tête et/ou du corps adoptée pour l'activité habituelle concernée, c'est-à-dire la position et/ou l'orientation de la tête et/ou du corps dans un référentiel associé à une cible visuelle fixée du regard par le porteur,

- l'ergorama associant à chaque direction du regard la distance entre l'œil et le point regardé,
- la variabilité de l'un des paramètres énoncé précédemment,
- la position de la monture de lunettes du porteur sur son visage.

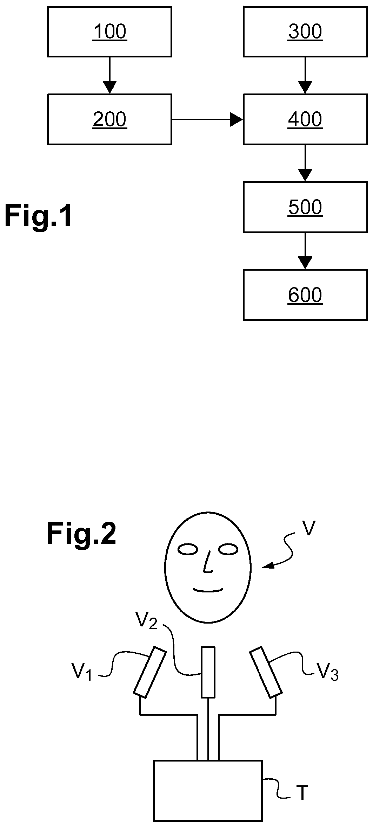

Selon l'invention, le procédé comporte les étapes suivantes (blocs 100 à 600 de la figure 1):

a) on réalise 100 l'acquisition d'un premier set de données correspondant à une première représentation de la tête du porteur en trois dimensions, grâce à un premier dispositif de capture d'image V1,
b) on détermine 200, à partir de ce set de données, la position relative dans l'espace d'au moins trois points particuliers morphologiques prédéfinis du visage du porteur,
c) on réalise 300 l'acquisition d'un deuxième set de données correspondant à une deuxième représentation de la tête du porteur dans une posture naturelle, comprenant au moins une image de chacun des trois points particuliers morphologiques prédéfinis du visage du porteur, grâce à un deuxième dispositif de capture d'image V2, distinct du premier dispositif de capture d'image V1,
d) on identifie 400, sur ladite deuxième représentation de l'étape c), l'image de chacun desdits points particuliers morphologiques prédéfinis du visage du porteur dont les positions relatives dans l'espace ont été déterminées à l'étape b),
e) on déduit 500 de cette identification des informations relatives à la position et/ou à l'orientation de la tête du porteur lors de la détermination de ladite deuxième représentation,
f) on détermine 600, à partir des informations déduites à l'étape e), ledit paramètre comportemental du porteur..

De préférence, à l'étape a), l'acquisition d'une première représentation de la tête du porteur est réalisée par une personne spécialisée dans l'optométrie, autre que le porteur, et/ou à l'aide d'un dispositif dédié à l'optométrie, tandis que, à l'étape c), l'acquisition d'une deuxième représentation de la tête du porteur est réalisée sous la responsabilité du porteur lui-même, sans intervention d'une personne spécialisée dans l'optométrie et/ou à l'aide d'un dispositif d'usage courant.

Ainsi, il est possible de découpler la détermination de la première représentation et celle de la deuxième représentation de la tête du porteur.

La détermination de la première représentation de la tête du porteur peut être réalisée chez un opticien, avec les moyens dédiés possédés par lui, et dans les conditions particulières associées au lieu de travail de l'opticien, notamment en termes de positionnement du porteur, de l'éclairage, des dispositifs de mesure et du temps court dédié à la mesure. En revanche, la deuxième représentation de la tête du porteur est déterminée de préférence par le porteur lui-même, dans son environnement, à l'aide de dispositifs de mesure d'usage courant, comme un appareil photographique, une caméra ou une webcam, avec la possibilité d'y consacrer un temps beaucoup plus long que celui dédié à cette détermination chez l'opticien.

En outre, dans le cas ou la détermination de chaque représentation comprend l'acquisition d'une ou plusieurs images par un dispositif de capture d'image, on comprendra que le premier dispositif de capture d'image associé à l'acquisition de la première représentation et le deuxième dispositif de capture d'image associé à l'acquisition de la deuxième représentation sont distincts.

De même, lorsque l'une de ces deux étapes comprend la simple récupération d'un set de données correspondant à la première et/ou deuxième représentation de la tête du porteur, chaque set de données contient de préférence les données numériques correspondant à une ou plusieurs captures d'image en deux ou trois dimensions, réalisées préalablement, avec un premier et un deuxième dispositifs de capture d'image distincts.

Ainsi, en pratique, selon une possibilité privilégiée de mise en oeuvre de l'invention, les étapes du procédé se déroulent globalement de la manière suivante.

Le porteur se rend chez l'opticien, il sélectionne une monture de lunettes. Le porteur peut alors choisir des lentilles ophtalmiques sans personnalisation.

L'étape a) de prise de mesure en trois dimensions est réalisée de préférence sur place, par exemple à l'aide de caméras stéréoscopiques ou d'un scanner 3D, par exemple. L'opticien peut également récupérer un set de données préexistant, correspondant par exemple à une prise de mesure effectuée antérieurement. Ce set de données peut également éventuellement être transmis au porteur.

Un modèle en trois dimensions de la tête du porteur est réalisé à l'étape b), soit chez l'opticien, soit dans un centre de calcul déporté après transmission des données acquises.

Afin de personnaliser les lentilles ophtalmiques destinées à être montées dans la monture choisie par le porteur, l'opticien demande au porteur de prendre des photos ou des vidéos de lui dans diverses situations de son quotidien.

Celui-ci utilise à cet effet des moyens de mesure grand public comme un appareil photographique, un caméscope, une caméra embarquée dans un smartphone, une tablette, un écran télé, une webcam, un module de Kinect...

De préférence, le porteur procède à l'étalonnage du moyen de mesure par prise d'image d'un étalon dédié.

Il réalise une ou plusieurs séries de mesures au quotidien. Le porteur se prends en photo dans des situations de son quotidien : au travail, en lecture, dans des situations de détente (télévision, jeux...), ou lors d'activités particulières (cuisine, sport, musique, voiture...).

Les séries de mesures peuvent être répétées plusieurs fois.

L'ensemble des données acquises sont transmises pour traitement à l'opticien ou à un centre de calcul distant.

L'association du modèle en trois dimensions de la tête du porteur aux données collectées par le porteur au quotidien permet de les traiter de façon statistique pour en extraire des paramètres comportementaux tels que :

- distance de fixation
- ergorama
- position de la monture actuelle.

Ces paramètres comportementaux sont utilisés pour personnaliser les lentilles ophtalmiques destinées au porteur. Ils permettent de modifier la lentille ophtalmique standard pour l'adapter au mieux aux besoins du porteur. Il est également possible de pondérer des paramètres comportementaux déjà déterminés chez l'opticien.

Les différentes étapes du procédé selon l'invention vont maintenant être décrites en détail.

Etape a)

Ainsi, en pratique, l'étape a) est de préférence réalisée par une personne spécialisée dans l'optométrie, par exemple, chez un opticien, ou dans un kiosque équipé d'un dispositif dédié à l'optométrie, connecté à un dispositif informatique.

L'étape a) a pour but de réaliser une prise de mesure permettant de réaliser un modèle en trois dimensions au moins de la tête du porteur. Un modèle en trois dimensions, partiel ou complet, du corps du porteur peut également être déterminé. Par exemple, un modèle du buste du porteur peut être réalisé à partir de la détermination de la position de deux points particuliers du buste, à savoir les points acromions nommés manubrium et apophyse xyphoïde.

Le manubrium désigne la partie supérieure du sternum, sur laquelle s'articulent les clavicules. L'apophyse xyphoïde est une structure osseuse ou cartilagineuse qui se situe à la partie inférieure du sternum. Il est ainsi possible de déterminer la position de la tête par rapport au buste.

Ce modèle en trois dimensions est réalisé à l'étape b), comme expliqué plus loin.

Cette prise de mesure peut être réalisée de différentes manières.

Selon un premier mode de réalisation de l'étape a), on réalise une acquisition d'au moins une image en trois dimensions de la tête du porteur grâce à un premier dispositif de capture d'image en trois dimensions, cette image en trois dimensions constituant alors ladite première représentation de la tête du porteur.

Le premier dispositif de capture d'image en trois dimensions est par exemple un dispositif du type scanner en trois dimensions, ou un dispositif de capture d'images stéréoscopiques comprenant au moins deux appareils de capture d'image en deux dimensions enregistrant simultanément deux images en deux dimensions de la tête du porteur dans deux plans de capture d'image orientés de manière différentes par rapport à la tête du porteur. Dans ce cas, chaque image en trois dimensions comprend au moins deux images en deux dimensions ainsi que les informations liées au positionnement relatif des deux appareils de capture d'image.

Le premier dispositif de capture d'image en trois dimensions peut notamment être basé sur une technique de lumière structurée.

Ce premier dispositif de capture comprend alors des moyens de projection de lumière structurée, comportant par exemple un motif tel qu'une figure de moiré, sur la tête de l'individu pendant que des moyens de capture d'image enregistrent une ou plusieurs images en deux dimensions de la tête de l'individu.

Le motif étant connu, le traitement de ces images permet de déterminer la représentation en trois dimensions.

On peut envisager, de préférence, de faire l'acquisition d'une série de premières images en trois dimensions, cette série constituant alors la première représentation de la tête du porteur.

Le porteur est de préférence ici tête nue, c'est-à-dire que sa tête est dépourvue d'équipement optique tel que monture de lunettes ou lentille ophtalmique.

En pratique, pour la mise en oeuvre de cette étape a), on peut prévoir le dispositif représenté sur la figure 2.

La figure 2 représente un système de prise de mesures sur le visage V de la tête du porteur. Ce système comprend notamment n caméras vidéo $V_i$ (n=3 dans le mode de réalisation présenté en figure 1) et un dispositif de traitement T relié à chacune des n caméras $V_i$.

Le dispositif de traitement T est par exemple basé sur une architecture à microprocesseur. Dans un tel système, le microprocesseur exécute des instructions d'un programme mémorisé dans une mémoire associée au microprocesseur afin de mettre en oeuvre des procédés de traitement comme ceux présentés ci-après.

Le dispositif de traitement comprend par exemple en outre un écran, une interface utilisateur (tel qu'un clavier ou une souris) et un appareil électronique de stockage, tel qu'un disque dur. Ces éléments sont connectés au microprocesseur et sont commandés par le microprocesseur du fait de l'exécution d'instructions dédiées par le microprocesseur.

Les caméras vidéo $V_i$ sont étalonnées et calibrées les unes par rapport aux autres, c'est-à-dire que, dans un plan de prise de vue au niveau duquel le porteur positionnera son visage, les n caméras vidéos $V_i$ acquiè-

rent des images représentant la même zone du visage du porteur.

Chaque caméra vidéo $V_i$ acquiert une séquence d'images bidimensionnelles $I_i(t_1)$, . . . , $I_i(t_m)$ prises respectivement à des instants $t_1,...,t_m$.

Selon un deuxième mode de réalisation de l'étape a), on réalise l'acquisition d'une série de premières images en deux dimensions de la tête du porteur disposée dans différentes postures à l'aide d'un premier dispositif de capture d'image en deux dimensions, cette série de premières images en deux dimensions constituant ladite première représentation de la tête du porteur, et on détermine, pour chaque première image en deux dimensions capturées, la position et/ou l'orientation correspondantes de la tête du porteur par rapport au premier dispositif de capture d'image.

On considère dans ce cas une série de premières images non stéréoscopiques : le premier dispositif de capture d'image capture, à un instant t donné, une seule image en deux dimensions de la tête du porteur.

On peut envisager à cet effet de placer sur la tête du porteur un accessoire présentant des caractéristiques géométriques déterminées. Il peut s'agir par exemple de la monture de lunettes choisie ou d'une monture autre que celle choisie, présentant de préférence des dimensions supérieures à celle choisie de manière à ce que le regard du porteur ne soit pas contraint par les bords de la monture.

La monture est de préférence équipée d'un système de repérage destiné à permettre la détermination de la position de la tête du porteur dans l'espace à partir d'une image capturée de la tête du porteur équipée du système de repérage. Ce système de repérage est décrit en détails dans le document FR2914173, page 7, ligne 5 à page 10, ligne 8. Il ne sera donc pas décrit plus en détail ici.

Ce système de repérage présente des caractéristiques géométriques prédéterminées, qui permettent, à partir d'une image capturée de la tête du porteur sur laquelle apparaît ce système de repérage, de déterminer la position et l'orientation de la tête du porteur dans l'espace, dans un référentiel lié au dispositif de capture d'image. Ce système de repérage permet donc de déterminer la position et l'orientation d'un référentiel lié à la tête du porteur dans le référentiel lié au dispositif de capture d'image.

La tête du porteur peut être directement équipée du système de repérage.

Selon un troisième mode de réalisation de l'étape a), on stocke ledit set de données comprenant ladite première représentation de la tête du porteur qui a été prédéterminée. L'étape a) ne comprend pas alors d'étape de capture d'image. Il s'agit de récupérer un fichier numérique prédéterminé.

Ce set de données peut notamment contenir les données correspondant à une image en trois dimensions capturées précédemment (par exemple à l'aide d'un scanner en trois dimensions ou d'un dispositif de capture d'image stéréoscopiques) ou à une série d'images en deux dimensions capturées précédemment. Dans ce dernier cas, le set contient également en association avec les données correspondant à chaque image en deux dimensions, des informations relatives à la position et/ou l'orientation de la tête du porteur par rapport au premier dispositif de capture d'image lors de la capture de cette image.

Etape b)

On détermine, à partir de ce set de données, la position relative dans l'espace d'au moins trois points particuliers de la tête du porteur.

De préférence, on détermine la position relative dans l'espace d'au moins quatre points particuliers de la tête du porteur.

Cette étape correspond à l'établissement d'un référentiel en trois dimensions associé à la tête du porteur. Ce référentiel en trois dimensions est un référentiel absolu, dans l'espace, ou lié à l'environnement du porteur, qui est non lié au premier dispositif de capture d'image.

Lesdits trois points particuliers de la tête du porteur peuvent être des points particuliers situés directement sur la tête du porteur.

Il s'agit alors de préférence de points morphologiques prédéfinis du visage, par exemple, les commissures des lèvres, l'extrémité des ailes du nez, les coins de chaque oeil du porteur, appelés les canthus internes et externes des yeux, les sourcils ou les dents de la mâchoire supérieure.

Quel que soit le mode de réalisation de l'étape a), le set de données établit à l'étape a) est stocké à cet effet dans un serveur informatique, qui peut éventuellement comprendre le dispositif de traitement T décrit précédemment. Le serveur informatique peut être localisé chez l'opticien ou déportée dans un centre de calcul distant. Dans ce dernier cas, le set de données est transmis au serveur informatique distant.

En pratique, à l'étape b), on effectue une étape de reconnaissance de l'image de chacun de ces points particuliers sur chaque image capturée à l'étape a) ou dans le set de données récupéré à l'étape a).

En particulier, dans le cas où l'on réalise, à l'étape a), l'acquisition d'une série de premières images en trois dimensions de la tête du porteur, alors on détermine, à l'étape b), grâce à une analyse statistique, lesdits points particuliers en fonction des points de la tête du porteur dont les positions relatives sont les plus stables sur la série de premières images en trois dimensions. Cette analyse statistique peut notamment comprendre la détermination d'un modèle statistique de la tête du porteur.

Par exemple, dans le cas où l'on capture, à l'étape a), une image en trois dimensions de la tête du porteur grâce à un dispositif de capture d'image stéréoscopique, les sous-étapes suivantes peuvent être mise en oeuvre à l'étape b). Le serveur informatique comprend ici le dispositif de traitement T décrit précédemment. Chacune des sous-étapes décrites ci-dessous est ici mise en oeuvre par le microprocesseur du dispositif de traitement T, sur la base d'instructions mémorisées dans une mémoire associée au microprocesseur ; les données traitées par le microprocesseur (telles que les images prises par les caméras ou les coordonnées des points caractéristiques dans les différents modèles) sont également mémorisées dans cette mémoire, ou stockées par un autre moyen, tel qu'un disque dur. Tout autre moyen de mise en oeuvre connu de l'homme du métier peut également être envisagé. Le traitement des données peut par exemple être réalisé par un hyperviseur d'un cluster de serveurs virtualisés.

Dans une première sous-étape, on détermine, dans chaque image $I_i(t_j)$, une pluralité de p points caractéristiques du visage représenté dans l'image concernée. On note $Q_{i,j}(1),...,Q_{i,j}(p)$ les p points déterminés comme correspondant dans une image $I_i(t_j)$ aux p points caractéristiques du visage. Le dispositif de traitement mémorise alors par exemple les coordonnées (bidimensionnelles) de chacun des points caractéristiques dans l'image concernée.

Cette détermination des points caractéristiques est par exemple réalisée à l'aide d'un algorithme de reconnaissance faciale, ici de type *"Active Appearance Models"* (voir par exemple à ce sujet l'article *"Active appearance models"*, de T.F. Cootes, G.J. Edwards, C.J. Taylor, in IEEE Transactions on Pattern Analysis and Machine Intelligence 23 (6): 681, 2011).

On procède ensuite à une deuxième sous-étape au cours de laquelle on associe les points caractéristiques de chaque série d'image $I_1(t_j),..., I_n(t_j)$, c'est-à-dire de chaque ensemble de n images prises par les n caméras $V_i$ à un même instant $t_j$, afin d'obtenir, pour chaque série d'images, un modèle tridimensionnel du visage, à savoir un ensemble de localisations des points caractéristiques du visage définies par des coordonnées tridimensionnelles.

Précisément, pour chaque point caractéristique k du visage et pour chaque série d'images $I_1(t_j),...,I_n(t_j)$ (correspondant aux images prises à un instant $t_j$), on utilise les coordonnées des points $Q_{1,j}(k),...,Q_{n,j}(k)$ dans ces images afin d'évaluer les coordonnées tridimensionnelles du point $P_k(t_j)$ où est situé le point caractéristique k du visage à l'instant $t_j$, par exemple en utilisant la géométrie épipolaire (et grâce à la calibration des caméras $V_i$ déjà mentionnée).

On remarque ici que, du fait que le visage du porteur est mobile et déformable, le modèle tridimensionnel obtenu (défini par l'ensemble des points $P_k(t_j)$ à un instant $t_j$) est a priori variable selon l'instant $t_j$ considéré.

On procède ensuite à une troisième sous-étape à laquelle on ajuste chaque modèle tridimensionnel obtenu à l'étape précédente par rapport à un modèle de référence.

On utilise par exemple le modèle tridimensionnel associé à l'instant ti comme modèle de référence lors de la première itération de la troisième sous-étape. En variante, on pourrait utiliser pour la première itération de la troisième sous-étape un autre modèle tridimensionnel obtenu à la deuxième sous-étape comme modèle de référence.

Pour chaque modèle tridimensionnel (associé à la prise de vue à l'instant $t_j$), l'étape d'ajustement est ici réalisée en minimisant la distance euclidienne entre le nuage de points du modèle tridimensionnel concerné et le nuage de points du modèle tridimensionnel de référence, par translation et rotation dans l'espace du nuage de points du modèle tridimensionnel concerné.

Ainsi, si on note $R_1,..., R_p$ les points du modèle de référence (pour la première itération, comme déjà indiqué,- on utilise le modèle associé à l'instant $t_1$, soit $R_k=P_k(t_1)$ pour k allant de 1 à p), on cherche donc la transformation F (composée d'une translation et d'une rotation) qui minimise la distance euclidienne entre le nuage de points $R_k$ du modèle de référence et le nuage de points du modèle concerné après transformation, c'est-à-dire qui minimise :

$$\sum_{k=1}^{p} d\left(F\left(P_k(t_j)\right), R_k\right)$$

, où d est la distance (euclidienne) entre deux points.

On note $P'_k(t_j)$ les points du modèle ajusté (i.e. après ajustement) :

$$P'_k(t_j) = F(P_k(t_j)).$$

La détermination des points du modèle ajusté (ou, ce qui est équivalent, de la transformation F) est par exemple mise en oeuvre au moyen d'un algorithme de type ICP (pour *"Iterative Closest Point"* - à ce sujet voir par exemple l'article *"Comparing ICP Variants on Real-World Data Sets"* de F. Pomerleau, F. Colas, R. Siegwart et S. Magnenat in Autonomous Robots, 34(3), pages 133-148, avril 2013.)

On procède alors à une quatrième sous-étape de construction d'un modèle statistique formé d'un ensemble de points $S_k$ sur la base des modèles ajustés, formés chacun des points $P'_k(t_j)$. Précisément, chaque point $S_k$ du modèle statistique est construit sur la base des points $P'_k(t_1),... ,P'_k(t_m)$ où est situé un point caractéristique donné k du visage aux différents instants $t_1,...,t_m$ de prise de vue.

Pour chaque point caractéristique k du visage, on détermine par exemple le point $S_k$ du modèle statistique comme l'isobarycentre des points $P'_k(t1),...,P'_k(t_m)$ correspondants dans les différents modèles ajustés.

En variante, on peut rejeter les points aberrants : pour définir $S_k$, on détermine à nouveau l'isobarycentre des points $P'_k(t_j)$, sans tenir compte toutefois des points trop éloignés du premier isobarycentre calculé (isobarycentre calculé sur la base des m points $P'_k(t_j)$), par exemple situés à une distance du premier isobarycentre calculé supérieure à un seuil. On propose ici d'utiliser comme seuil, dans la distribution des distances des différents points au premier isobarycentre calculé, la moyenne plus deux écarts-type.

En variante, le point $S_k$ du modèle statistique peut être construit comme le barycentre des points $P'_k(t_1),...,P'_k(t_m)$ pondérés par un coefficient lié par exemple :

- à l'erreur résiduelle du point après ajustement (c'est-à-dire la distance euclidienne entre le point $P'_k(t_j)$, après ajustement, concerné et le point de référence associé) ;
- à un coefficient d'erreur déterminé lors de la reconnaissance des points caractéristiques du visage à la deuxième sous-étape (la reconnaissance de certains points pouvant être plus ou moins certaine en fonction de la position du visage).

On propose ici par ailleurs de n'utiliser dans le modèle statistique que les points $S_k$ pour lesquels l'incertitude est faible. On utilise par exemple comme mesure de l'incertitude (pour chaque valeur de k) la somme de la moyenne et de deux fois l'écart-type de la distribution formée des distances entre le point $S_k$ déterminé comme indiqué ci-dessus et les différents points $P'_k(t_j)$ correspondants dans les modèles ajustés.

Seuls les points $S_k$ pour lesquels cette mesure d'incertitude est inférieure à un seuil prédéterminé sont conservés dans la suite du traitement. Si cette condition ne permet pas de conserver trois points, on conserve les trois points ayant la plus faible mesure d'incertitude.

Ceci permet de sélectionner les points particuliers les plus stables et les plus caractéristiques du visage traité et ainsi de renforcer la robustesse du modèle, qui pourra dès lors fournir un référentiel métrologique stable et précis.

En variante, on pourrait permettre à l'utilisateur (par exemple un opticien) de choisir (par sélection interactive, par exemple au moyen de l'écran et de l'interface utilisateur du dispositif de traitement T) les points $S_k$ qui sont le plus représentatifs, ou de donner une pondération à chacun des points $S_k$.

Selon une autre variante, on pourrait reprendre dans le modèle statistique l'ensemble des points $S_k$ déterminés chacun à partir des m points $P'_k(t_j)$ correspondants dans les m modèles ajustés.

Le modèle statistique obtenu lors de la première itération de la quatrième sous-étape peut être utilisé en tant que référentiel métrologique. Il est toutefois également possible de minimiser les erreurs d'ajustement comme expliqué à présent.

On détermine dans ce cas lors d'une cinquième sous-étape ultérieure si une nouvelle itération des troisième et quatrième sous-étapes est nécessaire. Pour ce faire, on calcule par exemple une fonction de mérite égale à la moyenne des mesures d'incertitudes obtenues pour l'ensemble des points $S_k$ conservés à la quatrième sous-étape.

S'il est déterminé lors de la cinquième sous-étape qu'une nouvelle itération des troisième et quatrième sous-étapes doit être mise en oeuvre (par exemple parce que la fonction de mérite qui vient d'être calculée est supérieure à un seuil prédéfini et qu'un nombre prédéterminé d'itérations n'a pas été atteint), le procédé se poursuit en retournant à la troisième sous-étape, en utilisant cette fois comme modèle de référence le modèle statistique obtenu lors de la dernière itération de la quatrième sous-étape. On met donc en oeuvre la troisième sous-étape en utilisant, pour les points $S_k$ pour lesquels l'incertitude est faible : = $S_k$.

S'il est déterminé lors de la cinquième sous-étape qu'une nouvelle itération des troisième et quatrième sous-étapes n'est pas nécessaire (par exemple par ce que la fonction de mérite qui vient d'être calculée est inférieure ou égale au seuil prédéfini ou que le nombre prédéterminé d'itérations est atteint), on peut utiliser le dernier modèle statistique obtenu.

Ainsi, on détermine au moins trois points particuliers $S_k$ associés à la tête du porteur, à partir de l'analyse statistique de la série de premières images en trois dimensions de la tête du porteur capturées à l'étape a), dans le cas de l'utilisation d'un premier dispositif de capture d'images stéréoscopique.

Les points particuliers de la tête du porteur peuvent également comprendre des points particuliers indirectement situés sur la tête du porteur.

### Etape c)

En pratique, l'étape c) est de préférence réalisée par le porteur, chez lui ou dans son environnement habituelle - bureau, voiture... - et à l'aide de dispositif de capture d'image d'usage courant.

L'étape c) a pour but de réaliser une prise de mesure en posture naturelle du porteur, c'est-à-dire dans une posture non contrainte.

De préférence, à l'étape c), la deuxième représentation de la tête du porteur est déterminée pendant une ou plusieurs activités habituelles du porteur, dans son environnement.

Plus précisément, l'activité habituelle du porteur peut notamment être l'une des activités suivantes :

- lecture sur différents supports,
- travail à un bureau,
- conduite automobile,
- pratique d'un sport,
- cuisine,
- jeu sur console de jeu,
- repos assis,
- regarder la télévision,
- jouer d'un instrument de musique.

Cette prise de mesure peut être réalisée de différentes manières.

Selon un premier mode de réalisation de l'étape c), à l'étape c), on réalise l'acquisition d'au moins une deuxième image en trois dimensions de la tête du porteur grâce à un deuxième dispositif de capture d'image en trois dimensions distinct du premier dispositif de capture d'image, ladite deuxième image en trois dimensions constituant ladite deuxième représentation de la tête du porteur.

Le deuxième dispositif de capture d'image en trois dimensions est par exemple un dispositif du type scanner en trois dimensions, ou un dispositif de capture d'images stéréoscopiques comprenant au moins deux appareils de capture d'image en deux dimensions enregistrant simultanément deux images en deux dimensions de la tête du porteur dans deux plans de capture d'image orientés de manière différentes par rapport à la tête du porteur. Dans ce cas, chaque image en trois dimensions comprend au moins deux images en deux dimensions ainsi que les informations liées au positionnement relatif des deux appareils de capture d'image.

Il peut s'agir notamment d'un dispositif du type « Kinect », dont le principe de fonctionnement est décrit dans le document US20100118123.

On peut envisager, de préférence, de faire l'acquisition d'une pluralité d'image en trois dimensions.

Le porteur est de préférence ici tête nue, c'est-à-dire que sa tête est dépourvue d'équipement optique tel que monture de lunettes ou lentille ophtalmique.

Selon un deuxième mode de réalisation de l'étape c), on capture, à l'aide d'un deuxième dispositif de capture d'image distinct du premier dispositif de capture d'image, au moins une deuxième image en deux dimensions de la tête du porteur constituant ladite deuxième représentation de la tête du porteur.

De préférence, selon un troisième mode de réalisation de l'étape c), on réalise l'acquisition d'une série de deuxièmes images en deux dimensions de la tête du porteur disposée dans différentes postures à l'aide d'un deuxième dispositif de capture d'image en deux dimensions, cette série de deuxièmes images constituant ladite deuxième représentation de la tête du porteur, et on détermine, pour chaque deuxième image capturée, la position et/ou l'orientation correspondantes de la tête du porteur par rapport au deuxième dispositif de capture d'image.

Comme décrit en référence à l'étape a), on peut envisager à cet effet de placer sur la tête du porteur un accessoire présentant des caractéristiques géométriques déterminées. Il peut s'agir par exemple de la monture de lunettes choisie ou d'une monture autre que celle choisie, présentant de préférence des dimensions supérieures à celle choisie de manière à ce que le regard du porteur ne soit pas contraint par les bords de la monture.

La monture est de préférence équipée d'un système de repérage destiné à permettre la détermination de la position de la tête du porteur dans l'espace à partir d'une image capturée de la tête du porteur équipée du système de repérage. Ce système de repérage est décrit en détails dans le document FR2914173, page 7, ligne 5 à page 10, ligne 8. Il ne sera donc pas décrit plus en détail ici.

Ce système de repérage présente des caractéristiques géométriques prédéterminées, qui permettent, à partir d'une image capturée de la tête du porteur sur laquelle apparaît ce système de repérage, de déterminer la position et l'orientation de la tête du porteur dans l'espace, dans un référentiel lié au dispositif de capture d'image. Ce système de repérage permet donc de déterminer la position et l'orientation d'un référentiel lié à la tête du porteur dans le référentiel lié au dispositif de capture d'image.

La tête du porteur peut être directement équipée du système de repérage.

Selon une variante de ce troisième mode de réalisation de l'étape c), la série de deuxièmes images de la tête du porteur étant acquise pendant que le porteur fixe du regard une cible visuelle, on détermine la position de cette cible visuelle par rapport au deuxième dispositif de capture d'image, lors de la capture de chaque deuxième image.

Ceci peut par exemple être réalisé grâce à un dispositif de suivi de la direction du regard du type oculomètre.

Un tel oculomètre est par exemple intégré à certains téléphones portables et par conséquent facilement accessible au grand public.

Une étape de calibration préalable de l'oculomètre peut être faite chez l'opticien ou chez le porteur.

En variante, on peut également prévoir que cette cible présente une position prédéterminée connue par rapport au deuxième dispositif de capture d'image utilisé à l'étape c). C'est le cas notamment lorsque ce deuxième dispositif de capture d'image est intégré à un écran d'affichage ou présente tout du moins une position fixe et prédéterminée par rapport à un écran d'affichage sur lequel est affichée la cible.

Il est également possible, à l'aide d'instructions données au porteur, de lui demander de placer le dispositif de capture d'image à un endroit précis, par exemple sur le tableau de bord en voiture ou sur la télévision ou l'écran regardé par le porteur.

Par ailleurs, quel que soit le mode de réalisation de l'étape c) mis en oeuvre, on détermine de préférence en outre la position et/ou l'orientation dans l'espace dudit deuxième dispositif de capture d'image lors de la capture de ladite deuxième image en deux ou trois dimensions, et on en déduit des informations relatives à la position et/ou à l'orientation de la tête du porteur dans l'espace lors de la capture de cette deuxième image.

On peut à cet effet prévoir de réaliser une étape de calibration du deuxième dispositif de capture d'image en deux ou trois dimensions, au cours de laquelle on capture, avec ce deuxième dispositif de capture d'image, une image d'un objet étalon dédié.

Cet objet étalon est par exemple une image représentant une mire, par exemple un damier en noir et blanc de dimensions connues. Cet objet étalon est placé à la distance de travail du porteur, par exemple sur le support de lecture (livre, écran d'ordination, tablette, etc...) utilisé par le porteur. Une image de cet objet étalon est capturée à l'aide du deuxième dispositif de capture d'image utilisé par le porteur.

L'objet étalon peut être affiché par une application sur un téléphone portable, notamment sur un smartphone. Ce téléphone portable peut également aussi servir à la transmission des sets de données et permettre l'association à ces set de données de l'identifiant du porteur.

L'application de ce téléphone portable peut également être utilisée pour calibrer la webcam d'un ordinateur en plaçant le téléphone en face de cette webcam. En mettant le téléphone portable et l'ordinateur « face à face », ou plutôt « écran à écran », leurs caméras respectives peuvent se calibrer. Les dispositifs de capture de l'ordinateur et du téléphone portable peuvent se reconnaitre (marque et modèle).

On peut aussi déterminer les caractéristiques d'un appareil photo en photographiant une mire que l'application du téléphone portable affiche.

Cela permet de déterminer ultérieurement, par traitement de cette image de l'objet étalon, la position et l'orientation du deuxième dispositif de capture d'image par rapport à cet objet étalon, donc par rapport au support de lecture.

On enregistre par ailleurs, de préférence, pour chaque deuxième image capturée, les informations relatives à la configuration du deuxième dispositif de capture d'image utilisé à l'étape c). Il s'agit par exemple des informations donnant la distance focale de l'objectif de ce dispositif, l'ouverture, etc... Ces informations permettent de déterminer la distance entre le porteur et le deuxième dispositif de capture d'image au moment de la capture de la deuxième image concernée, et/ou la distance entre la cible et le deuxième dispositif de capture d'image, selon les informations présentes dans la deuxième image capturée.

Par ailleurs, le deuxième dispositif de capture d'image peut comporter des moyens de mesure, comme par exemple un gyromètre, présent de manière habituelle dans les téléphones portables équipés d'un appareil photographique, qui peuvent permettre de déterminer l'orientation dans l'espace du deuxième dispositif de capture d'image. Les mesures relevées par ces moyens de mesure pendant la capture d'une image sont également enregistrées.

De manière générale, toutes les informations relatives à la capture de la deuxième image sont stockées en association avec cette deuxième image.

Selon un quatrième mode de réalisation de l'étape c), on stocke ledit set de données comprenant ladite deuxième représentation de la tête du porteur qui a été prédéterminée.

Ce set de données peut notamment contenir les données correspondant à une ou plusieurs images en trois ou en deux dimensions capturées précédemment. Dans le cas de plusieurs images en deux dimensions, le set peut contenir également, en association, avec les données correspondant à chaque image en deux dimensions, des informations relatives à la position et/ou l'orientation de la tête du porteur par rapport au deuxième dispositif de capture d'image lors de la capture de cette image.

En pratique, comme décrit précédemment, l'étape c) est réalisée dans un lieu différent du lieu de réalisation de l'étape a).

Quel que soit le mode de réalisation de l'étape c), le set de données établit à l'étape c) est transmis et stocké dans le serveur informatique, qui peut éventuellement comprendre le dispositif de traitement T décrit précédemment.

De préférence, on envisage que, dans une étape préliminaire à l'étape d), chaque première image en deux ou trois dimensions capturée à l'étape a) est transmise à un serveur informatique et stockée en correspondance avec un identifiant du porteur. L'identifiant peut être associé au porteur ou à l'équipement du porteur. Par équipement on entend notamment une monture ancienne ou nouvellement choisie.

Après la réalisation de l'étape b), les trois points particuliers déterminés sont également transmis au serveur informatique et stockés en correspondance avec cet identifiant.

De la même façon, chaque deuxième image en deux ou trois dimensions capturée à l'étape c) est transmise à un serveur informatique et stockée en correspondance avec cet identifiant du porteur.

Ainsi, chaque deuxième image capturée transmise au serveur peut être associée avec les points particuliers de la tête du porteur déterminés à l'étape a) et stockée sur le serveur en correspondance avec ledit identifiant du porteur.

Etape d)

A l'étape d), on identifie, sur ladite deuxième représentation de l'étape b), l'image de chacun desdits points particuliers de la tête du porteur dont les positions relatives dans l'espace ont été déterminées à l'étape b).

Il s'agit alors d'effectuer une reconnaissance d'image qui peut être par exemple réalisée grâce à un algorithme de reconnaissance faciale, ici de type *"Active Appearance Models"* (voir par exemple à ce sujet l'article *"*Active appearance models", de T.F. Cootes, G.J. Edwards, C.J. Taylor, in IEEE *Transactions on Pattern Analysis and Machine Intelligence 23 (6): 681, 2011).

Etape e)

A l'étape e), on déduit de cette identification des informations relatives à la position et/ou à l'orientation de la tête du porteur lors de la détermination de ladite deuxième représentation.

A cet effet, on recherche la position et/ou l'orientation du référentiel lié à la tête du porteur défini par les trois points particuliers identifiés à l'étape b) lors de la capture des deuxièmes images de la deuxième représentation ou lors de la détermination des données contenues dans le set correspondant.

Ainsi, à l'étape e), on déduit lesdites informations relatives à la position et/ou à l'orientation de la tête du porteur lors de la détermination de ladite deuxième représentation à partir de l'identification réalisée à l'étape d) et de la position relative dans l'espace des trois points particuliers de la tête du porteur déterminée à l'étape b).

De manière générale, à l'étape e), on détermine par calcul la position et/ou l'orientation de la tête du porteur lors de la détermination de ladite deuxième représentation, comme étant la position et/ou l'orientation de la tête du porteur pour laquelle les trois points particuliers de la tête du porteur présenteraient les coordonnées des images des trois points particuliers identifiés sur ladite deuxième représentation. Il s'agit d'un calcul d'optimisation de la position et de l'orientation du référentiel de la tête du porteur défini par les trois points particuliers identifiés à l'étape b).

Il s'agit en fait de projeter le référentiel en trois dimensions de la tête du porteur déterminé à l'étape b) sur chaque deuxième image, de manière à déterminer la position et/ou l'orientation de la tête du porteur au moment de la capture de cette deuxième image dans un référentiel lié au deuxième dispositif de capture d'image.

La méthode appelée « méthode Poslt », publiée par Daniel F. DeMenthon et Larry S. Davis en mai 1995 peut être utilisée dans ce but. Cette méthode permet de trouver la position et/ l'orientation d'un objet à partir d'une seule image en deux dimensions et d'un modèle de l'objet en trois dimensions.

La mise en oeuvre de cette méthode nécessite de trouver au moins 4 points de correspondance entre l'objet en deux dimensions et le modèle en trois dimensions.

La méthode se décompose en deux étapes. Une première étape correspond à la projection du modèle en trois dimensions sur l'image en deux dimensions et à la détermination d'une matrice de rotation et d'un vecteur de translation permettant de faire coïncider les points particuliers du modèle et les images de ces points particuliers identifiés sur les deuxièmes images en deux dimensions.

La deuxième étape est une étape d'itération utilisant un facteur d'échelle pour améliorer la précision de la projection réalisée à la première étape.

Selon une autre possibilité simplifiée, par exemple, dans le cas où :

- à l'étape a), on capture une série de premières images en deux dimensions et on détermine, pour chaque première image capturée, la position et/ou l'orientation correspondantes de la tête du porteur par rapport au premier dispositif de capture d'image ;

- à l'étape c), on capture, à l'aide d'un deuxième dispositif de capture d'image distinct du premier dispositif de capture d'image, au moins une deuxième image en deux dimensions de la tête du porteur constituant ladite deuxième représentation de la tête du porteur,

alors, à l'étape e), on associe à la deuxième image capturée à l'étape c) la première image de ladite série de premières images la plus proche de ladite deuxième image et on identifie la position et/ou l'orientation de la tête du porteur par rapport audit deuxième dispositif de capture d'image lors de la capture de ladite deuxième image à la position et/ou l'orientation de la tête du porteur lors de la capture de cette première image la plus proche.

Pour ce faire, on détermine la moyenne des distances euclidiennes entre les points particuliers de chaque première image de la série de premières images et les points particuliers correspondants de la deuxième image considérée.

On associe à la deuxième image capturée à l'étape c) la première image de la série de premières images pour laquelle cette moyenne est la plus faible. Par ailleurs, lorsque la position et/ou l'orientation du deuxième dispositif de capture d'image peut être déterminée dans un référentiel absolu de l'espace grâce aux moyens de mesure embarqués du deuxième dispositif de capture d'image (gyromètre), il est alors possible de déduire la position et/ou l'orientation de la tête du porteur dans un tel référentiel absolu de l'espace.

Etape f)

A l'étape f), on détermine, à partir de ladite deuxième représentation et des informations déduites à l'étape e), ledit paramètre comportemental du porteur.

A cet effet, dans le cas où, à l'étape c), on réalise l'acquisition d'une série de deuxièmes images de la tête du porteur, à l'étape f), on détermine ledit paramètre comportemental du porteur en fonction d'un traitement statistique desdites deuxièmes images de cette série. On remarque que cela est possible grâce à la construction d'un référentiel de la tête du porteur à l'étape b) et à la détermination subséquente de la position et/ou de l'orientation de la tête du porteur à l'étape e) ; la détermination de l'étape f) est réalisée en tenant compte de cette position et/ou de cette orientation.

Par exemple, dans le cas où le porteur réalise l'étape c) pendant une tâche visuelle de lecture sur un écran, grâce à un deuxième dispositif de capture d'image solidaire de cet écran, il est possible de déterminer, à l'étape e), pour chaque deuxième image de la tête du porteur, la distance entre le dispositif de capture d'image et le porteur. Cette distance peut être assimilée à la distance de lecture lorsque le dispositif de capture d'image est porté par le support de lecture.

Il est également possible de déduire cette distance de lecture des deuxièmes images capturées à l'étape c) et de la position et/ou de l'orientation de la tête du porteur déterminée à l'étape e).

Ceci peut être réalisé de différente manière, soit par traitement des images capturées, lorsque le porteur est équipé d'un système de repérage, soit par utilisation des données issues d'une étape préalable de calibration du deuxième dispositif de capture d'image, soit par utilisation des informations relatives à la configuration de ce deuxième dispositif pendant la capture d'image (notamment distance focale).

La distance de lecture peut alors être déterminée comme une moyenne arithmétique des distances de lecture déterminées pour l'ensemble ou une partie des deuxièmes images de la série de deuxièmes images.

Le paramètre comportemental recherché peut également comprendre un coefficient oeil-tête égal au rapport entre l'amplitude du déplacement d'un oeil du porteur selon une direction déterminée et l'amplitude maximale du déplacement de cet oeil pendant une tâche en vision de près, par exemple une tâche de lecture.

Dans ce contexte, les étapes a) et b), réalisées tel que décrites précédemment, servent à déterminer une posture de référence du porteur.

Les étapes c) à e) sont également réalisées comme décrit précédemment.

En outre, à l'étape c), on place le porteur dans une situation dans laquelle il effectue une tâche de lecture prédéfinie.

Cette tâche de lecture implique la lecture d'au moins un paragraphe comportant plusieurs lignes, par exemple 4 ou 5 lignes, de gauche à droite ou de droite à gauche selon la langue concernée.

On constate que le porteur prend une position plus naturelle au fur et à mesure de la lecture, notamment lorsqu'il change de page.

On utilise donc de préférence un texte comportant plusieurs pages, et on exploite plus particulièrement les images capturées correspondant aux dernières pages lues.

Le texte constitue alors une cible dont la position est connue à tout instant par rapport au support. Elle est donc connue dans le référentiel lié au deuxième dispositif de capture d'image, par exemple un dispositif de type téléphone intelligent (ou *"smartphone"* selon l'appellation anglo-saxonne couramment utilisée).

A l'étape c), on réalise alors, à l'aide du deuxième dispositif de capture d'image, une capture d'image de la tête du porteur pour différentes positions de la cible correspondant à différentes directions de regard à la première distance de travail.

A partir de ces deuxièmes images capturées, le dispositif de traitement du serveur informatique détermine par exemple la position du centre de rotation CRO d'au moins un oeil du porteur dans le référentiel lié à la tête du porteur. Le principe de cette détermination est connu en soi et exposé par exemple dans le document FR2914173, dont un équivalent en anglais est le document US20100128220.

En variante, les positions du centre de rotation de l'œil dans le référentiel lié à la tête du porteur, de la pupille de l'œil du porteur, le rayon de l'œil du porteur peuvent être prédéterminés, par exemple par une mesure faite par l'opticien.

Ils peuvent être intégrés au set de données desdites deuxièmes images et constituer les paramètres de calibration du deuxième dispositif de capture d'image.

A l'étape d), on identifie, sur ladite deuxième représentation de l'étape b), l'image de chacun desdits points particuliers de la tête du porteur dont les positions relatives dans l'espace ont été déterminées à l'étape b), et à l'étape e), on déduit de cette identification des informations relatives à la position et/ou à l'orientation de la tête du porteur lors de la détermination de ladite deuxième représentation.

En pratique, à l'étape e), on détermine la posture de la tête du porteur pour chaque deuxième image capturée.

Il est alors possible de déterminer la direction du regard du porteur dans le référentiel lié à la tête du porteur lors de chaque capture d'image comme étant la droite reliant ce centre de rotation avec la cible dans sa position correspondante pendant la capture d'image.

On en déduit, à l'étape f), à partir de ladite deuxième représentation et des informations déduites à l'étape e), l'amplitude angulaire du déplacement des yeux du porteur lors de la tâche de lecture, comme étant l'écart angulaire entre les deux directions de regard les plus éloignées, dans un plan donné, notamment horizontal ou vertical. Cela revient à comparer toutes les postures de la tête du porteur sur les deuxièmes images capturées par rapport à cette référence.

Connaissant les dimensions du texte affiché sur le support et lu par le porteur, ainsi que la distance de lecture du porteur, c'est-à-dire la distance entre les yeux du porteur et ce texte, on détermine alors l'amplitude angulaire maximale du déplacement de chaque oeil comme étant l'étendue angulaire du texte vu par le porteur, selon la direction horizontale ou verticale considérée.

En divisant l'amplitude angulaire du mouvement de chaque oeil par l'amplitude angulaire maximale du déplacement de chaque oeil, on en déduit un coefficient appelé coefficient oeil-tête caractéristique du comportement du porteur lors d'une tâche de lecture.

Ce coefficient quantifie la propension du porteur à bouger les yeux ou la tête lors de la tâche de lecture. Il est également possible de déterminer la moyenne de l'amplitude angulaire du déplacement de l'œil comme étant la moyenne des déplacements angulaire de l'œil gauche et de l'œil droit du porteur. On peut alors en déduire un coefficient œil-tête moyen.

La posture statique ou dynamique adoptée pour l'activité habituelle concernée correspond par exemple à l'orientation, c'est-à-dire l'inclinaison de la tête, par rapport à un élément associé à l'activité concernée du porteur : support de lecture, instrument de musique, tableau de bord d'un véhicule...

L'ergorama associant à chaque direction du regard la distance entre l'œil et le point regardé peut être déterminé à partir des directions du regard déterminée comme exposé précédemment, en faisant varier la distance de travail, par exemple la distance de lecture, entre deux captures d'une deuxième image. Un traitement statistique des séries de deuxièmes images en deux ou trois dimensions capturées permet également de déduire une grandeur caractérisant la variabilité des paramètres comportementaux énoncés précédemment.

Une faible variance assure que le paramètre comportemental caractérise bien le porteur. Une forte variance pourrait signifier que le porteur peut être difficilement caractérisé par ce paramètre.

Ainsi, un paramètre comportemental présentant une faible variance sera avantageusement pris en compte pour la conception de la lentille ophtalmique destinée au porteur, tandis qu'un paramètre comportemental présentant une forte variance ne sera pas pris en compte pour la conception de la lentille ophtalmique.

Enfin le paramètre comportemental déterminé peut être la position de la monture de lunettes du porteur sur son visage.

Il est en effet possible de déterminer la position de points remarquables de la monture dans le référentiel de la tête du porteur constitués à partir des points particuliers identifiés. La position de la monture de lunettes peut ainsi être déterminée dans ce référentiel de la tête du porteur.

Cette position peut comprendre par exemple une position relative des branches et des oreilles du porteur, et/ou une position des cercles de la monture par rapport aux pupilles du porteur et/ou une position du pontet de la monture par rapport au nez du porteur. La position du pontet de la monture peut être déterminée selon une direction verticale, correspondant à la hauteur sur le nez du porteur et en profondeur correspondant à une distance verre-oeil.

**Revendications**

1. Procédé de détermination d'au moins un paramètre comportemental d'un porteur en vue de la conception d'une lentille ophtalmique pour une monture de lunettes de ce porteur, ledit paramètre comportemental étant l'un des suivants :

   - distance de lecture,
   - rapport des déplacements angulaires de l'oeil et de la tête,
   - posture statique ou dynamique adoptée pour l'activité habituelle concernée,
   - ergorama associant à chaque direction du regard la distance entre l'œil et le point regardé,
   - variabilité des paramètres comportementaux énoncés précédemment,
   - position de la monture de lunettes du porteur sur son visage, et ledit procédé comportant les étapes suivantes :

   a) on réalise (100) l'acquisition d'un premier set de données correspondant à une première représentation de la tête du porteur en trois dimensions, grâce à un premier dispositif de capture d'image (V1),
   b) on détermine (200), à partir de ce set de données, la position relative dans l'espace d'au moins trois points particuliers morphologiques prédéfinis du visage du porteur,
   c) on réalise (300) l'acquisition d'un deuxième set de données correspondant à une deuxième représentation de la tête du porteur dans une posture naturelle, comprenant au moins une image de chacun des trois points particuliers morphologiques prédéfinis du visage du porteur, grâce à un deuxième dispositif de capture d'image (V2), distinct du premier dispositif de capture d'image (V1),
   d) on identifie (400), sur ladite deuxième représentation de l'étape c), l'image de chacun desdits points particuliers morphologiques prédéfinis du visage du porteur dont les positions relatives dans l'espace ont été déterminées à l'étape b),
   e) on déduit (500) de cette identification des informations relatives à la position et/ou à l'orientation de la tête du porteur lors de la détermination de ladite deuxième représentation,
   f) on détermine (600), à partir des informations déduites à l'étape e), ledit paramètre comportemental du porteur.

2. Procédé selon la revendication 1, selon lequel, à l'étape a), ledit premier dispositif de capture d'image étant un dispositif de capture d'image en trois dimensions, on réalise une acquisition d'au moins une image en trois dimensions de la tête du porteur grâce audit premier dispositif de capture d'image en trois dimensions, cette image en trois dimensions constituant alors ladite première représentation de la tête du porteur.

3. Procédé selon la revendication 2, selon lequel, à l'étape a), on réalise l'acquisition d'une série de premières images en trois dimensions de la tête du porteur et à l'étape b), on détermine, grâce à une analyse statistique, lesdits points particuliers en fonction des points de la tête du porteur dont les positions relatives sont les plus stables sur la série de premières images en trois dimensions.

4. Procédé selon la revendication 1, selon lequel,

- à l'étape a), ledit premier dispositif de capture d'image étant un dispositif de capture d'image en deux dimensions, on réalise l'acquisition d'une série de premières images en deux dimensions de la tête du porteur disposée dans différentes postures à l'aide dudit premier dispositif de capture d'image en deux dimensions, cette série de premières images constituant ladite première représentation de la tête du porteur, et on détermine, pour chaque première image capturée, la position et/ou l'orientation correspondantes de la tête du porteur par rapport au premier dispositif de capture d'image.

5. Procédé selon la revendication précédente, selon lequel,

- à l'étape c), on capture, à l'aide dudit deuxième dispositif de capture d'image distinct du premier dispositif de capture d'image, au moins une deuxième image en deux dimensions de la tête du porteur constituant ladite deuxième représentation de la tête du porteur,
- à l'étape e), on associe à la deuxième image capturée à l'étape c) la première image de ladite série de premières images la plus proche de ladite deuxième image et on identifie la position et/ou l'orientation de la tête du porteur par rapport audit deuxième dispositif de capture d'image lors de la capture de ladite deuxième image à la position et/ou l'orientation de la tête du porteur lors de la capture de cette première image la plus proche.

6. Procédé selon la revendication 1, selon lequel,

- à l'étape a) et/ou à l'étape c), on stocke ledit set de données comprenant lesdites première et deuxième représentations de la tête du porteur qui ont été prédéterminées.

7. Procédé selon l'une des revendications précédentes, selon lequel, à l'étape c), la deuxième représentation de la tête du porteur est déterminée pendant une ou plusieurs activités habituelles du porteur, dans son environnement.

8. Procédé selon l'une des revendications précédentes, selon lequel, à l'étape c), on capture, à l'aide dudit deuxième dispositif de capture d'image, au moins une deuxième image en deux dimensions de la tête du porteur constituant ladite deuxième représentation de la tête du porteur.

9. Procédé selon l'une des revendications 1 à 7, selon lequel, à l'étape c), ledit deuxième dispositif de capture d'image étant un dispositif de capture d'image en trois dimensions, on réalise l'acquisition d'au moins une deuxième image en trois dimensions de la tête du porteur grâce audit deuxième dispositif de capture d'image en trois dimensions, ladite deuxième image en trois dimensions constituant ladite deuxième représentation de la tête du porteur.

10. Procédé selon l'une des revendications 8 et 9, selon lequel, à l'étape c), on réalise l'acquisition d'une série de deuxièmes images en deux ou trois dimensions de la tête du porteur et, à l'étape f), on détermine ledit paramètre comportemental du porteur en fonction d'un traitement statistique desdites deuxièmes images de cette série.

11. Procédé selon la revendication précédente, selon lequel, à l'étape c), la série de deuxièmes images de la tête du porteur étant acquise pendant que le porteur fixe du regard une cible visuelle, on détermine la position de cette cible visuelle par rapport au deuxième dispositif de capture d'image, lors de la capture de chaque deuxième image.

12. Procédé selon l'une des revendications 8 à 11, selon lequel on réalise une étape de calibration du deuxième dispositif de capture d'image en deux ou trois dimensions, au cours de laquelle on capture, avec ce deuxième dispositif de capture d'image, une image d'un objet étalon dédié.

13. Procédé selon l'une des revendications 8 à 12, selon lequel on détermine en outre la position et/ou l'orientation dans l'espace dudit deuxième dispositif de capture d'image lors de la capture de ladite deuxième image en deux ou trois dimensions, et on en déduit des informations relatives à la position et/ou à l'orientation de la tête du porteur dans l'espace lors de la capture de cette deuxième image.

14. Procédé selon l'une des revendications 8 à 13, selon lequel, dans une étape préliminaire à l'étape d), chaque deuxième image en deux ou trois dimensions capturée à l'étape b) est transmise à un serveur informatique et stockée en correspondance avec un identifiant du porteur.

15. Procédé selon la revendication 8 à 14, selon lequel chaque deuxième image capturée transmise au serveur est associée avec les points particuliers morphologiques du porteur déterminés à l'étape a) et stockée sur le serveur en correspondance avec ledit identifiant du porteur.

16. Procédé selon l'une des revendications 1 à 15, selon lequel, à l'étape e), on détermine par calcul la position et/ou l'orientation de la tête du porteur lors de la détermination de ladite deuxième représentation, comme étant la position et/ou l'orientation de la tête du porteur pour laquelle les trois points particuliers morphologiques du porteur présenteraient les coordonnées des images des trois points particuliers identifiés sur ladite deuxième représentation.

17. Procédé selon l'une des revendications 1 à 16, selon lequel, à l'étape a), l'acquisition d'une première représentation de la tête du porteur est réalisée par une personne spécialisée dans l'optométrie, autre que le porteur, et/ou à l'aide d'un dispositif dédié à l'optométrie, tandis que, à l'étape c), l'acquisition d'une deuxième représentation de la tête du porteur est réalisée sous la responsabilité du porteur lui-même, sans intervention d'une personne spécialisée dans l'optométrie et/ou à l'aide d'un dispositif d'usage courant.

**Patentansprüche**

1. Verfahren zur Bestimmung mindestens eines Verhaltensparameters eines Trägers im Hinblick auf die Auslegung einer ophthalmischen Linse für ein Brillengestell dieses Trägers, wobei der Verhaltensparameter einer der folgenden ist:

- Leseabstand,
- Verhältnis zwischen den winkelförmigen Bewegungen des Auges und des Kopfes,
- statische oder dynamische Haltung, die bei der betroffenen typischen Aktivität eingenommen wird,
- Ergorama, das jeder Blickrichtung den Abstand zwischen dem Auge und dem betrachteten Punkt zuordnet,
- Variabilität der zuvor genannten Verhaltensparameter,
- Position des Brillengestells des Trägers auf seinem Gesicht, und wobei das Verfahren die folgenden Schritte umfasst:

a) Durchführen (100) der Erfassung eines ersten Datensatzes, der einer ersten Darstellung des Kopfes des Trägers in drei Dimensionen entspricht, mittels einer ersten Bildaufnahmevorrichtung (V1),
b) Bestimmen (200), anhand dieses Datensatzes, der relativen Position im Raum von mindestens drei vordefinierten bestimmten morphologischen Punkten des Gesichts des Trägers,
c) Durchführen (300) der Erfassung eines zweiten Datensatzes, der einer zweiten Darstellung des Kopfes des Trägers in einer natürlichen Haltung entspricht und mindestens ein Bild von jedem der drei vordefinierten bestimmten morphologischen Punkte des Gesichts des Trägers beinhaltet, mittels einer zweiten Bildaufnahmevorrichtung (V2), die sich von der ersten Bildaufnahmevorrichtung (V1) unterscheidet,
d) Identifizieren (400), in der zweiten Darstellung des Schritts c), des Bildes von jedem der vordefinierten bestimmten morphologischen Punkte des Gesichts des Trägers, von denen die relativen Positionen im Raum in Schritt b) bestimmt wurden,
e) Ableiten (500), aus dieser Identifizierung, von Informationen bezüglich der Position und/oder der Ausrichtung des Kopfes des Trägers während der Bestimmung der zweiten Darstellung,
f) Bestimmen (600), anhand der in Schritt e) abgeleiteten Informationen, des Verhaltensparameters des Trägers.

2. Verfahren nach Anspruch 1, wobei in Schritt a), wenn die erste Bildaufnahmevorrichtung eine Vorrichtung zur dreidimensionalen Bildaufnahme ist, eine Erfassung mindestens eines dreidimensionalen Bildes des Kopfes des Trägers mittels der ersten Vorrichtung zur dreidimensionalen Bildaufnahme durchgeführt wird, wobei dieses dreidimensionale Bild dann die erste Darstellung des Kopfes des Trägers bildet.

3. Verfahren nach Anspruch 2, wobei in Schritt a) die Erfassung einer Reihe von ersten dreidimensionalen Bildern des Kopfes des Trägers durchgeführt wird und in Schritt b) mittels einer statistischen Analyse die bestimmten Punkte in Abhängigkeit von den Punkten des Kopfes des Trägers bestimmt werden, von denen die relativen Positionen in der Reihe von ersten dreidimensionalen Bildern am stabilsten sind.

4. Verfahren nach Anspruch 1, wobei

- in Schritt a), wenn die erste Bildaufnahmevorrichtung eine Vorrichtung zur zweidimensionalen Bildaufnahme ist, die Erfassung einer Reihe von ersten zweidimensionalen Bildern des Kopfes des Trägers, der in unterschiedlichen Haltungen angeordnet ist, mit Hilfe der ersten Vorrichtung zur zweidimensionalen Bildaufnahme durchgeführt wird, wobei diese Reihe von ersten Bildern die erste Darstellung des Kopfes des Trägers bildet, und für jedes aufgenommene erste Bild die entsprechende Position und/oder Ausrichtung des Kopfes des Trägers im Verhältnis zu der ersten Bildaufnahmevorrichtung bestimmt wird.

5. Verfahren nach dem vorhergehenden Anspruch, wobei

- in Schritt c) mit Hilfe der zweiten Bildaufnahmevorrichtung, die sich von der ersten Bildaufnahmevorrichtung unterscheidet, mindestens ein zweites zweidimensionales Bild des Kopfes des Trägers aufgenommen wird, das die zweite Darstellung des Kopfes des Trägers bildet,
- in Schritt e) dem in Schritt c) aufgenommenen zweiten Bild das erste Bild der Reihe von ersten Bildern, das dem zweiten Bild am nächsten kommt, zugeordnet wird und die Position und/oder Ausrichtung des Kopfes des Trägers im Verhältnis zu der zweiten Bildaufnahmevorrichtung während der Aufnahme des zweiten Bildes in der Position und/oder Ausrichtung des Kopfes des Trägers während der Aufnahme dieses ersten, am nächsten kommenden Bildes identifiziert wird.

6. Verfahren nach Anspruch 1, wobei

- in Schritt a) und/oder in Schritt c) der Datensatz, der die erste und zweite Darstellung des Kopfes des Trägers, die zuvor bestimmt wurden, beinhaltet, gespeichert wird.

7. Verfahren nach einem der vorhergehenden Ansprüche, wobei in Schritt c) die zweite Darstellung des Kopfes des Trägers während einer oder mehrerer typischer Aktivitäten des Trägers in seiner Umgebung bestimmt wird.

8. Verfahren nach einem der vorhergehenden Ansprüche, wobei in Schritt c) mit Hilfe der zweiten Bildaufnahmevorrichtung mindestens ein zweites zweidimensionales Bild des Kopfes des Trägers aufgenommen wird, das die zweite Darstellung des Kopfes des Trägers bildet.

9. Verfahren nach einem der Ansprüche 1 bis 7, wobei in Schritt c), wenn die zweite Bildaufnahmevorrichtung eine Vorrichtung zur dreidimensionalen Bildaufnahme ist, die Erfassung mindestens eines zweiten dreidimensionalen Bildes des Kopfes des Trägers mittels der zweiten Vorrichtung zur dreidimensionalen Bildaufnahme durchgeführt wird, wobei dieses zweite dreidimensionale Bild dann die zweite Darstellung des Kopfes des Trägers bildet.

10. Verfahren nach einem der Ansprüche 8 und 9, wobei in Schritt c) die Erfassung einer Reihe von zweiten zwei-oder dreidimensionalen Bildern des Kopfes des Trägers durchgeführt wird und in Schritt f) der Verhaltensparameter des Trägers in Abhängigkeit von einer statistischen Verarbeitung der zweiten Bilder dieser Reihe bestimmt wird.

11. Verfahren nach dem vorhergehenden Anspruch, wobei in Schritt c), wenn die Reihe von zweiten Bildern des Kopfes des Trägers erfasst wird, während der Träger ein visuelles Ziel mit dem Blick fixiert, die Position dieses visuellen Ziels im Verhältnis zu der zweiten Bildaufnahmevorrichtung während der Aufnahme jedes zweiten Bildes bestimmt wird.

12. Verfahren nach einem der Ansprüche 8 bis 11, wobei ein Schritt des Kalibrierens der zweiten Vorrichtung zur zwei- oder dreidimensionalen Bildaufnahme durchgeführt wird, während dessen mit dieser zweiten Bildaufnahmevorrichtung ein Bild eines speziellen Referenzobjekts aufgenommen wird.

13. Verfahren nach einem der Ansprüche 8 bis 12, wobei ferner die Position und/oder Ausrichtung im Raum der zweiten Bildaufnahmevorrichtung während der Aufnahme des zweiten zwei- oder dreidimensionalen Bildes bestimmt wird und daraus Informationen bezüglich der Position und/oder der Ausrichtung des Kopfes des Trägers im Raum während der Aufnahme dieses zweiten Bildes abgeleitet werden.

14. Verfahren nach einem der Ansprüche 8 bis 13, wobei in einem Schritt vor dem Schritt d) jedes zweite zwei- oder dreidimensionale Bild, das in Schritt b) aufgenommen wurde, an einen Computerserver übertragen und zusammen mit einer Kennung des Trägers gespeichert wird.

15. Verfahren nach Anspruch 8 bis 14, wobei jedes aufgenommene zweite Bild, das an den Server übertragen wird, den bestimmten morphologischen Punkten des Trägers, die in Schritt a) bestimmt wurden, zugeordnet wird und zusammen mit der Kennung des Trägers auf dem Server gespeichert wird.

16. Verfahren nach einem der Ansprüche 1 bis 15, wobei in Schritt e) durch Berechnen die Position und/oder Ausrichtung des Kopfes des Trägers während der Bestimmung der zweiten Darstellung als die Position und/oder Ausrichtung des Kopfes des Trägers bestimmt wird, für die die drei bestimmten morphologischen Punkte des Trägers die Koordinaten der Bilder der drei bestimmten Punkte aufweisen, die in der zweiten Darstellung identifiziert wurden.

17. Verfahren nach einem der Ansprüche 1 bis 16, wobei in Schritt a) die Erfassung einer ersten Darstellung des Kopfes des Trägers durch einen Optometriespezialisten, der nicht der Träger ist, und/oder mit Hilfe einer speziellen Optometrievorrichtung durchgeführt wird, während in Schritt c) die Erfassung einer zweiten Darstellung des Kopfes des Trägers unter der Verantwortung des Trägers selber durchgeführt wird, ohne dass ein Optometriespezialist daran mitwirkt und/oder mit Hilfe einer Vorrichtung des täglichen Gebrauchs.

**Claims**

1. Method for determining at least one behavioural parameter of a wearer with a view to designing an ophthalmic lens for a spectacle frame of this wearer, said behavioural parameter being one of the following:

    - reading distance
    - ratio of the angular movements of the eye and head,
    - static or dynamic posture adopted for the habitual activity in question,
    - ergorama associating with each direction of the gaze the distance between the eye and the point looked at,
    - variability in the aforementioned behavioural parameters,
    - position of the spectacle frame of the wearer on his face, and said method including the following steps:

        a) acquiring (100) a first dataset corresponding to a first representation of the head of the wearer in three dimensions, by virtue of a first image-capturing device (V1),
        b) determining (200), from this dataset, the relative position in space of at least three particular predefined morphological points of the face of the wearer,
        c) acquiring (300) a second dataset corresponding to a second representation of the head of the wearer in a natural posture, comprising at least one image of each of the three particular predefined morphological points of the face of the wearer, by virtue of a second image-capturing device (V2) distinct from the first image-capturing device (V1),
        d) identifying (400), in said second representation of step c), the image of each of said particular predefined morphological points of the face of the wearer the relative positions in space of which were determined in step b),
        e) deducing (500) from this identification information relating to the position and/or orientation of the head of the wearer during the determination of said second representation,
        f) determining (600), from the information deduced in step e), said behavioural parameter of the wearer.

2. Method according to Claim 1, wherein, in step a), said first image-capturing device being a device for capturing three-dimensional images, at least one three-dimensional image of the head of the wearer is acquired by virtue of said first device for capturing three-dimensional images, this three-dimensional image then constituting said first representation of the head of the wearer.

3. Method according to Claim 2, wherein, in step a), a series of three-dimensional first images of the head of the wearer is acquired and, in step b), said particular points are determined, by virtue of a statistical analysis, depending on the points of the head of the wearer the relative positions of which are the most stable in the series of three-dimensional first images.

4. Method according to Claim 1, wherein,

    - in step a), said first image-capturing device being a device for capturing two-dimensional images, a series of two-dimensional first images of the head of the wearer in various cephalic postures is acquired using said first device for capturing two-dimensional images, this series of first images constituting said first representation of the head of the wearer, and, for each captured first image, the corresponding position and/or orientation of the head of the wearer with respect to the first image-capturing device is determined.

5. Method according to the preceding claim, wherein,

    - in step c), at least one two-dimensional second image of the head of the wearer is captured using said second image-capturing device that is distinct from the first image-capturing device, said at least one two-dimensional second image of the head of the wearer constituting said second representation of the head of the wearer,
    - in step e), the first image of said series of first images which is closest said second image is associated with the second image captured in step c) and the position and/or orientation of the head of the wearer with respect to said second image-capturing device during the capture of said second image is identified with the position and/or orientation of the head of the wearer during the capture of this closest first image.

6. Method according to Claim 1, wherein,

    - in step a) and/or in step c), said dataset comprising said first and second representations of the head of the wearer, which representations are determined beforehand, is stored.

7. Method according to one of the preceding claims, wherein, in step c), the second representation of the head of the wearer is determined during one or more habitual activities of the wearer, in his environment.

**8.** Method according to one of the preceding claims, wherein, in step c), at least one two-dimensional second image of the head of the wearer is captured using said second image-capturing device, said at least one two-dimensional second image of the head of the wearer constituting said second representation of the head of the wearer.

**9.** Method according to one of Claims 1 to 7, wherein, in step c), said second image-capturing device being a device for capturing three-dimensional images, at least one three-dimensional second image of the head of the wearer is acquired by virtue of said second device for capturing three-dimensional images, said three-dimensional second image constituting said second representation of the head of the wearer.

**10.** Method according to one of Claims 8 and 9, wherein, in step c), a series of two- or three-dimensional second images of the head of the wearer is acquired and, in step f), said behavioural parameter of the wearer is determined depending on a statistical treatment of said second images of this series.

**11.** Method according to the preceding claim, wherein, in step c), the series of second images of the head of the wearer being acquired while the wearer is fixating his gaze on a visual target, the position of this visual target with respect to the second image-capturing device is determined during the capture of each second image.

**12.** Method according to one of Claims 8 to 11, wherein a step of calibrating the second device for capturing two- or three-dimensional images is carried out, in which step an image of a dedicated standard object is captured with this second image-capturing device.

**13.** Method according to one of Claims 8 to 12, wherein the position and/or orientation in space of said second image-capturing device is further determined during the capture of said two- or three-dimensional second image, and information relating to the position and/or orientation of the head of the wearer in space during the capture of this second image is deduced therefrom.

**14.** Method according to one of Claims 8 to 13, wherein, in a step prior to step d), each two- or three-dimensional second image captured in step b) is transmitted to an information-processing server and stored in correspondence with an identifier of the wearer.

**15.** Method according to Claim 8 to 14, wherein each captured second image transmitted to the server is associated with those particular morphological points of the wearer which were determined in step a), said image being stored on the server in correspondence with said identifier of the wearer.

**16.** Method according to one of Claims 1 to 15, wherein, in step e), the position and/or orientation of the head of the wearer during the determination of said second representation are/is determined computationally as being the position and/or orientation of the head of the wearer for which the three particular morphological points of the wearer have the coordinates of the images of the three particular points identified in said second representation.

**17.** Method according to one of Claims 1 to 16, wherein, in step a), a first representation of the head of the wearer is acquired by a person specialized in optometry, other than the wearer, and/or using a device dedicated to optometry, whereas, in step c), a second representation of the head of the wearer is acquired under the responsibility of the wearer himself, without intervention of a person specialized in optometry and/or using a commonplace device.

**Fig.1**

**Fig.2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014016502 A1
- WO 0209025 A1
- FR 2987918 A1
- FR 2974722 A1

- FR 2914173
- US 20100118123 A
- US 20100128220 A

**Littérature non-brevet citée dans la description**

- **T.F. COOTES ; G.J. EDWARDS ; C.J. TAYLOR.** Active appearance models. *IEEE Transactions on Pattern Analysis and Machine Intelligence,* 2011, vol. 23 (6), 681
- **F. POMERLEAU ; F. COLAS ; R. SIEGWART ; S. MAGNENAT.** Comparing ICP Variants on Real-World Data Sets. *Autonomous Robots,* Avril 2013, vol. 34 (3), 133-148

- **DE T.F. COOTES ; G.J. EDWARDS ; C.J. TAYLOR.** Active appearance models. *IEEE *Transactions on Pattern Analysis and Machine Intelligence,* 2011, vol. 23 (6), 681